# EUROPÄISCHE PATENTSCHRIFT

(11) **EP 0 294 647 B1**
(45) Veröffentlichungstag und Bekanntmachung des Hinweises auf die Patenterteilung: **21.07.1993**
(21) Anmeldenummer: 88108308.3
(22) Anmeldetag: 25.05.1988
(51) Int. Cl.: C07D 417/04, A61K 31/54

(54) **Thiadiazinone**
Thiadiazinones
Thiadiazinones

(30) Priorität: 06.06.1987 DE 3719031; 24.12.1987 DE 3744149
(43) Veröffentlichungstag der Anmeldung: 14.12.1988
(73) Patentinhaber: MERCK PATENT GmbH, D-64271 Darmstadt (DE)
(72) Erfinder: Jonas, Rochus, Dr., D-6100 Darmstadt (DE); Piulats, Jaime, Dr, 08030 Barcelona (ES); Lues, Inge, Dr., D-6100 Darmstadt (DE); Klockow, Michael, Dr., D-6101 Rossdorf (DE)

(56) Entgegenhaltungen:
- EP-A- 0 052 442
- EP-A- 0 085 227
- EP-A- 0 180 158

## Beschreibung

Die Erfindung betrifft neue Thiadiazinone der Formel I 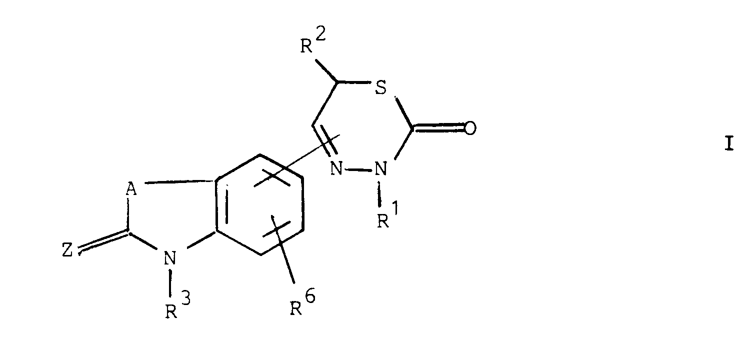
worin
- A: -CHR⁴-CHR⁵-, -CH₂-CR⁴R⁵-, CR⁴R⁵-CH₂-, -CHR⁴-CHR⁵-CH₂-, -CHR⁴-CH₂-CHR⁵-, -CH₂-CHR⁴-CHR⁵, -CR⁴R⁵-CH₂CH₂-, -CH₂-CR⁴R⁵-CH₂- oder -CH₂-CH₂-CR⁴R⁵-,
- R¹, R², R⁴ und R⁵: jeweils H, Alkyl, Alkenyl oder Alkinyl,
- R³: Alkenyl, Alkinyl oder Acyl mit bis zu 15 C-Atomen, ausgenommen unsubstituierte Alkanoyl-Reste
- R⁶: H, Alkyl, Alkoxy, OH, F, Cl, Br oder J und
- Z: (H, H), (H, Alkyl), (Alkyl, Alkyl) oder O bedeuten,
wobei die Alkyl-, Alkenyl-, Alkinyl- und/oder Alkoxy-gruppen jeweils bis zu 5 C-Atome enthalten, worin ferner, falls A -CH₂CH₂- und Z O bedeutet, einer der Reste R¹, R², R³ und R⁶ von H verschieden sein muß, sowie deren Salze.

Eine der Formel I ähnliche Verbindung, worin jedoch A = -CH₂CH₂-, Z = O und R¹, R², R³ und R⁶ jeweils = H sind, ist aus der EP-A-0180158 bekannt.

Darüber hinaus sind in EP 00 52 442 unter anderem 5-Phenyl-thiadiazinone mit verschiedenen Substituenten am Phenyl-Rest beschrieben. Die dort angegebene allgemeine Formel beansprucht auch durch 1,2,3,4-Tetrahydrochinolinyl-Reste substituierte Thiadiazinone, die beispielsweise am Tetrahydrochinolin unsubstituiert oder durch Oxo-, Alkyl- oder Alkanoyl-Reste substituiert sein können.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I bei guter Verträglichkeit wertvolle pharmakologische Eigenschaften besitzen. Insbesondere zeigen sie eine Wirkung auf die Herzkraft (positiv inotrope Wirksamkeit); ferner wirken die Substanzen vasodilatierend und fördern daher die Durchblutung. Die vasodilatierende und die Herzwirkung kann z. B. an narkotisierten oder wachen Hunden, Katzen, Affen oder Minischweinen, die positiv inotrope Wirkung auch an isolierten Herzpräparationen (z. B. Vorhof, Papillarmuskel oder perfundiertes Ganzherz) von Ratte, Meerschweinchen, Katze oder Hund ermittelt werden, z. B. nach Methoden, wie sie in Arzneimittelforschung, Band 31 (I) Nr. 1a (1981), Seiten 141 bis 170, oder von Schliep et al. im 9th International Congress of Pharmacol., London, Abstracts of papers 9P, beschrieben sind.

Weiterhin treten antithrombotische, thrombozytenaggregationshemmende und die Erythrozytenform beeinflussende Eigenschaften auf. Die Beeinflussung der Thrombozytenfunktion im Sinne einer Aggregationshemmung kann an der Ratte ex vivo im Test nach Born (Nature 194, 927-929, 1962) nachgewiesen werden. Die antithrombotische Wirkung zeigt sich in der Verlängerung der Blutungszeit nach Stella (Thrombos. Res. 7, 709-716, 1975), in der Verminderung des Thrombusgewichtes bei der kälteinduzierten Thrombosierung der Jugular-Vene bei der Ratte nach Meng (Ther. Ber. 47, 69-79, 1975) und der Erhöhung der zur vollständigen Thrombosierung notwendigen Laserimpulse an der Mensenterial-Venole der Ratte, entsprechend einer Abwandlung der Methode nach Kovacs (Microvasc. Res. 6, 194-201, 1973).

Die günstige Wirkung auf die Erythrozytenverformbarkeit ist im Nucleoporefilter nach Schmid-Schönbein (Pflüger's Archiv 338, 93-114, 1973) nachweisbar. Auch lassen sich günstige Effekte auf die Fibrinolyse/Euglobulinlysiszeit nach v. Kaulla (Progr. Chem. Fibrinol, Thrombol. 1, 131-149, 1975; ed J.F. Davidson, Raven Press, N.Y.) feststellen.

Die Verbindungen können daher als Arzneimittelwirkstoffe in der Human- und Veterinärmedizin verwendet werden. Ferner konnen sie als Zwischenprodukte zur Herstellung weiterer Arzneimittelwirkstoffe verwendet werden.

Gegenstand der Erfindung sind dementsprechend die Verbindungen der Formel I sowie ein Verfahren zu ihrer Herstellung, dadurch gekennzeichnet, daß man ein Keton der Formel II 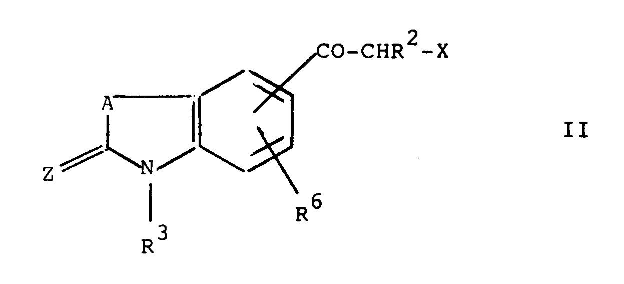
worin
- X: Cl, Br, J oder eine reaktionsfähig veresterte OH-Gruppe bedeutet und
- A, R², R³, R⁶ und Z: die angegebenen Bedeutungen haben,
mit einer Verbindung der Formel III

H₂N-NR¹-CS-OR III

worin
- R: Alkyl mit 1-5 C-Atomen oder ein Äquivalent eines Metall- oder Ammoniumkations
bedeutet und
- R¹: die angegebene Bedeutung hat
umsetzt
und/oder daß man gegebenenfalls eine Verbindung der Formel I, worin R¹ und/oder R³ H bedeuten, mit einem Alkylierungs-, Alkenylierungs-, Alkinylierungs- oder Acylierungsmittel behandelt und/oder eine Base der Formel I durch Behandeln mit einer Säure in eines ihrer Salze umwandelt.

Vor- und nachstehend haben A, R¹ bis R⁶, Z, X und R die bei den Formeln I, II bzw. III angegebenen Bedeutungen, wenn nicht ausdrücklich etwas anderes angegeben ist.

In den Formeln ist Alkyl vorzugsweise unverzweigt, hat vorzugsweise 1, 2 oder 3 C-Atome und steht bevorzugt für Methyl, ferner bevorzugt für Ethyl oder Propyl, weiterhin bevorzugt für Isopropyl, Butyl, Isobutyl, sek.-Butyl tert.-Butyl, n-Pentyl oder Isopentyl. Alkenyl ist vorzugsweise unverzweigt, hat vorzugsweise 2 oder 3 C-Atome und steht bevorzugt für Allyl, ferner bevorzugt für Vinyl oder Propen-1-yl. Alkinyl ist vorzugsweise unverzweigt, hat vorzugsweise 2 oder 3 C-Atome und steht bevorzugt für Propargyl, ferner bevorzugt für Ethinyl oder Propin-1-yl.

Acyl bedeutet den Säurerest einer Carbon- oder Sulfonsäure, vorzugsweise unsubstituiertes oder substituiertes Alkanoyl, Alkenoyl oder Alkinoyl mit 1-10, insbesondere 1, 2, 3, 4 oder 5 C-Atomen, im einzelnen bevorzugt Acetyl, ferner bevorzugt Formyl, Propionyl, Butyryl, Isobutyryl, Valeryl, Isovaleryl, Pivaloyl (Trimethylacetyl), Acryloyl, Hydroxyacetyl, Methoxyacetyl, Chloracetyl, Aminoacetyl (Glycyl), N-Methylaminoacetyl, N,N-Dimethylaminoacetyl, N,N-Diethylaminoacetyl, Pyrrolidinoacetyl, Piperidinoacetyl; weiterhin bevorzugt unsubstituiertes oder substituiertes Aroyl mit 7-15 C-Atomen, wobei als Substituenten insbesondere 1-3, vorzugsweise eine der folgenden Gruppen in Frage kommen: Alkyl, Alkoxy, Alkylthio, Alkylsulfinyl oder Alkylsulfonyl mit jeweils 1-3, vorzugsweise 1 oder 2 C-Atomen, Methylendioxy, ferner OH, F, Cl, Br, J, NO₂, NH₂, Alkylamino oder Dialkylamino mit jeweils 1-3, vorzugsweise 1 oder 2 C-Atomen in der Alkylgruppe. Einzelne bevorzugte Aroylreste sind Benzoyl, o-, m- oder p-Toluyl, o-, m- oder p-Methoxybenzoyl, 2,3-, 2,4-, 2,5-, 2,6-, 3,4- oder 3,5-Dimethoxybenzoyl, 2,3,4-, 2,3,5-, 2,3,6-, 2,4,5-, 2,4,6- oder 3,4,5-Trimethoxybenzoyl, o-, m- oder p-Methylthiobenzoyl, o-, m- oder p-Methylsulfinylbenzoyl, o-, m- oder p-Methylsulfonylbenzoyl, 2,3- oder 3,4-Methylendioxybenzoyl, o-, m- oder p-Fluorbenzoyl, o-, m- oder p-Chlorbenzoyl, 2- oder 3-Methoxy-4-methylthio-benzoyl, 2- oder 3-Methoxy-4-methylsulfinyl-benzoyl, o-, m- oder p-Dimethylaminobenzoyl, 2- oder 3-Methoxy-4-(2-hydroxyethoxy)-benzoyl, 2- oder 3-Methoxy-4-(2-methoxyethoxy)-benzoyl, 1- oder 2-Naphthoyl. Acyl kann weiterhin für Heterocyclylcarbonyl mit 2-10 C-Atomen wie 2- oder 3-Furoyl, 2- oder 3-Thenoyl, Isoxazolyl-4-carbonyl, 3-Phenyl-5-methyl-isoxazolyl-4-carbonyl, 1,5-Dimethyl-pyrazolyl-3-carbonyl, 2-Methyl-thiazolyl-4- oder -5-carbonyl, 1-Imidazolylcarbonyl, Picolinoyl, Nicotinoyl, Isonicotinoyl, 1-Methyl-2-, -3- oder -4-piperidinylcarbonyl stehen, außerdem für Arylalkanoyl wie Phenylacetyl, o-, m- oder p-Methoxyphenylacetyl, 2- oder 3-Phenylpropionyl, 2-, 3- oder 4-Phenylbutyryl; für Cycloalkylcarbonyl wie Cycloproylcarbonyl, Cyclopentylcarbonyl, Cyclohexylcarbonyl; für Alkylsulfonyl wie Methyl-, Ethyl-, Propyl- oder Butylsulfonyl; für Arylsulfonyl wie Benzolsulfonyl, o-, m- oder p-Toluolsulfonyl, o-, m- oder p-Methoxybenzolsulfonyl, 1- oder 2-Naphthalinsulfonyl.

Weitere besonders bevorzugte Acylgruppen leiten sich ab von der Kohlensäure und deren Estern sowie von der Carbaminsäure und deren N-Alkyl-, N,N-Dialkyl-, N-Aryl- und N-Alkanoylderivaten. Vorzugsweise seien als Beispiele solcher Acylgruppen genannt: Alkoxycarbonyl wie Methoxycarbonyl, Ethoxycarbonyl, Propoxycarbonyl, Isopropoxycarbonyl, Butoxycarbonyl oder Isobutoxycarbonyl; fluoriertes Alkoxycarbonyl wie 2,2,2-Trifluorethoxycarbonyl; Cycloalkoxycarbonyl wie Cyclopropoxycarbonyl, Cyclobutoxycarbonyl, Cyclopentoxycarbonyl oder Cyclohexoxycarbonyl; Aryloxycarbonyl wie Phenoxycarbonyl, o-, m- oder p-Methoxyphenoxycarbonyl; Aminocarbonyl; N-Alkylaminocarbonyl wie N-Methyl- oder N-Ethylaminocarbonyl; N,N-Dialkylaminocarbonyl wie N,N-Dimethyl- oder N,N-Diethylaminocarbonyl; N-Cycloalkylaminocarbonyl wie N-Cyclopropylaminocarbonyl, N-Cyclobutylaminocarbonyl, N-Cyclopentylaminocarbonyl, N-Cyclohexylaminocarbonyl; N,N-Dicycloalkylaminocarbonyl wie N,N-Dicyclopropylaminocarbonyl; Pyrrolidinocarbonyl; Piperidinocarbonyl; N-Arylaminocarbonyl wie Anilinocarbonyl; Alkanoylaminocarbonyl wie Formylaminocarbonyl, Acetylaminocarbonyl, Propionylaminocarbonyl, Butyrylaminocarbonyl, Isobutyrylaminocarbonyl.

Alkoxy ist vorzugsweise unverzweigt, hat vorzugsweise 1, 2 oder 3 C-Atome und steht bevorzugt für Methoxy, ferner bevorzugt für Ethoxy oder Propoxy, weiterhin z. B. für Isopropoxy, Butoxy, Isobutoxy, sek.-Butoxy, tert.-Butoxy, Pentoxy oder Isopentoxy.

Der Dihydrothiadiazinon-Ring steht vorzugsweise in der 6-Stellung, weiterhin bevorzugt in der 7-Stellung des Tetrahydroisochinolin-Rings, sowie vorzugsweise in der 7-Stellung, weiterhin bevorzugt in der 8-Stellung des Tetrahydrobenzazepin-Rings; er kann aber auch in der 5- oder 8-Stellung des Tetrahydroisochinolin- oder in der 6- oder 9-Stellung des Tetrahydrobenzazepin-Rings stehen.

Die Reste R¹, R², R³, R⁴, R⁵ und R⁶ bedeuten vorzugsweise jeweils H oder Methyl. Im einzelnen haben sie die folgenden bevorzugten Bedeutungen: R¹ H oder Methyl; R² Methyl; R³ H, Methoxycarbonyl, Ethoxycarbonyl, ferner auch Formyl, Acetyl oder Propionyl; R⁴ H oder Methyl; R⁵ H oder Methyl;
A ist vorzugsweise -CH₂CR⁴R⁵-, insbesondere -CH₂CH₂- oder -CH₂(CH₃)₂-, oder -CH₂CH₂-CR⁴R⁵-, insbesondere -CH₂CH₂CH₂- oder -CH₂CH₂C(CH₃)₂.

Der Rest Z ist vorzugsweise (H, H), besonders, wenn gleichzeitig der Rest R³ Acyl bedeutet, oder O, besonders, wenn gleichzeitig der Rest R³ H, Alkyl, Alkenyl oder Alkinyl bedeutet.

Gegenstand der Erfindung sind insbesondere Tetrahydrochinolinyl-1,3,4-thiadiazin-2-on-derivate der Formel I, worin A -CHR⁴-CHR⁵-, -CH₂-CR⁴R⁵- oder -CR⁴R⁵-CH₂- bedeutet ("Formel Ia") und Tetrahydrobenzazepinyl-1,3,4-thiadiazin-2-on-derivate der Formel I, worin A -CHR⁴-CHR⁵-CH₂-, -CHR⁴-CH₂-CHR⁵-, -CH₂-CHR⁴-CHR⁵, CR⁴R⁵-CH₂CH₂-, -CH₂-CR⁴R⁵-CH₂- oder -CH₂CH₂-CR⁴R⁵ bedeutet ("Formel Ib"). Bevorzugt sind diejenigen Verbindungen der Formeln I, Ia und Ib, in denen mindestens einer der genannten Reste eine der vorstehend angegebenen bevorzugten Bedeutungen hat. Einige bevorzugte Gruppen von Verbindungen der Formeln Ia bzw. Ib können durch die folgenden Teilformeln Iaa bis Iad bzw. Iba bis Ibd ausgedrückt werden, die den Formeln Ia bzw. Ib entsprechen und worin die nicht näher bezeichneten Reste die bei den Formeln I, Ia oder Ib angegebene Bedeutung haben, worin jedoch
in Iaa der Dihydrothiadiazinon-Ring in 6-Stellung steht,
- R¹: H,
- R², R³, R⁴ und R⁵: jeweils H oder Methyl,
- R⁶: H und
- Z: O bedeuten;
in Iab der Dihydrothiadiazinon-Ring in 6- oder 7-Stellung steht,
- R¹: H oder Alkyl mit 1-3 C-Atomen,
- R², R⁴ und R⁵: jeweils H oder Methyl,
- R³: Acyl mit 1-10 C-Atomen,
- R⁶: H und
- Z: (H,H) bedeuten;
in Iac der Dihydrothiadiazinon-Ring in 6- oder 7-Stellung steht,
- R¹: H oder Alkyl mit 1-3 C-Atomen,
- R², R⁴ und R⁵: jeweils H oder Methyl,
- R³: Alkanoyl mit 1-6 C-Atomen, Benzoyl, Toluyl, Methoxybenzoyl, Dimethoxybenzoyl, Methylthiobenzoyl, Methylsulfinyl-benzoyl, Methylsulfonyl-benzoyl, Picolinoyl, Nicotinoyl, Isonicotinoyl, Alkylsulfonyl mit 1-6 C-Atomen, Benzolsulfonyl oder Toluolsulfonyl,
- R⁶: H und
- Z: (H,H) bedeuten;
in Iad der Dihydrothiadiazinon-Ring in 6- oder 7-Stellung steht,
- R¹: H oder Alkyl mit 1-3 C-Atomen,
- R², R⁴ und R⁵: jeweils H oder Methyl,
- R³: Alkanoyl mit 1-5 C-Atomen,
- R⁶: H und
- Z: (H,H) bedeuten;
in Iba der Dihydrothiadiazinon-Ring in 7-Stellung steht,
- R¹: H,
- R², R³, R⁴ und R⁵: jeweils H oder Methyl,
- R⁶: H und
- Z: O bedeuten;
in Ibb der Dihydrothiadiazinon-Ring in 7- oder 8-Stellung steht,
- R¹: H oder Alkyl mit 1-3 C-Atomen,
- R², R⁴ und R⁵: jeweils H oder Methyl,
- R³: Acyl mit 1-10 C-Atomen,
- R⁶: H und
- Z: (H,H) bedeuten;
in Ibc der Dihydrothiadiazinon-Ring in 7- oder 8-Stellung steht,
- R¹: H oder Alkyl mit 1-3 C-Atomen,
- R², R⁴ und R⁵: jeweils H oder Methyl,
- R³: Alkanoyl mit 1-6 C-Atomen, Dialkylaminoalkanoyl mit 4-10 C-Atomen, Cyclohexylcarbonyl, Benzoyl, Toluyl, Methoxybenzoyl, Dimethoxybenzoyl, Methylthio-benzoyl, Methylsulfinyl-benzoyl, Methylsulfonyl-benzoyl, Fluorbenzoyl, Methoxymethylthiobenzoyl, 2- oder 3-Thenoyl, Picolinoyl, Nicotinoyl, Isonicotinoyl, Alkylsulfonyl mit 1-6 C-Atomen, Benzolsulfonyl, Toluolsulfonyl, Alkoxycarbonyl mit 2-6 C-Atomen, Phenoxycarbonyl, Aminocarbonyl, Alkylaminocarbonyl mit 2-6 C-Atomen, Dialkylaminocarbonyl mit 3-9 C-Atomen oder Alkanoylaminocarbonyl mit 2-6 C-Atomen,
- R⁶: H und
- Z: (H,H) bedeuten;
in Ibd der Dihydrothiadiazinon-Ring in 7-Stellung steht,
- R¹, R⁴, R⁵ und R⁶: jeweils H,
- R²: Methyl,
- R³: Cyclohexylcarbonyl, Methoxybenzoyl, Dimethoxybenzoyl, Fluorbenzoyl, 2-Methoxy-4-methylthiobenzoyl, 2-Thenoyl, Isonicotinoyl, Methoxycarbonyl, Ethoxycarbonyl oder Phenoxycarbonyl und
- Z: (H,H) bedeuten.

Die Verbindungen der Formel I werden im übrigen nach an sich bekannten Methoden hergestellt, wie sie in der Literatur (z. B. in den Standardwerken wie Houben-Weyl, Methoden der Organischen Chemie, Georg-Thieme-Verlag, Stuttgart; insbesondere in der EP-A-0 180 158) beschrieben sind, und zwar unter Reaktionsbedingungen, die für die genannten Umsetzungen bekannt und geeignet sind. Dabei kann man auch von an sich bekannten, hier nicht naher erwähnten Varianten Gebrauch machen.

In den Verbindungen der Formel II steht X bevorzugt für Cl oder Br. Falls X eine reaktionsfähig veresterte OH-Gruppe bedeutet, so ist diese vorzugsweise Alkylsulfonyloxy mit 1-6 C-Atomen, z. B. Methansulfonyloxy, oder Arylsulfonyloxy mit 6-10 C-Atomen, z. B. Benzol-, p-Toluol- oder 1- oder 2-Naphthalinsulfonyloxy.

In den Verbindungen der Formel II steht R bevorzugt für Methyl oder Ethyl, aber auch für Na, K oder NH₄.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die Ausgangsstoffe der Formeln II und III sind teilweise bekannt. Sofern sie nicht bekannt sind, können sie nach an sich bekannten Methoden hergestellt werden. Die Ketone der Formel II sind beispielsweise durch Friedel-Crafts-Synthese aus entsprechenden Tetrahydrochinolin- bzw. Tetrahydrobenzazepinderivaten mit Verbindungen der Formel X-CO-CHR²-X zugänglich.

Im einzelnen erfolgt die Umsetzung der Ketone der Formel II mit den Verbindungen der Formel III in Gegenwart oder Abwesenheit eines inerten Lösungsmittels bei Temperaturen zwischen etwa -20 und etwa +150°, vorzugsweise zwischen 20 und 100°. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; Alkohole wie Methanol, Ethanol oder Isopropanol; Glykole und Glykolether wie Ethylenglykol, Diethylenglykol, 2-Methoxyethanol; Nitrile wie Acetonitril; Ether wie Tetrahydrofuran oder Dioxan; Amide wie Dimethylformamid (DMF); Sulfoxide wie Dimethylsulfoxid. Auch Gemische dieser Lösungsmittel sind geeignet.

Eine Verbindung der Formel I, worin R¹ und/oder R³ H bedeuten, kann, falls erwünscht, alkyliert, alkenyliert, alkinyliert oder acyliert werden, wobei man die entsprechende Verbindungen der Formel I, worin R¹ und/oder R³ Alkyl, Alkenyl oder Alkinyl bzw. R³ auch Acyl bedeuten, erhält.

Als Alkylierungs-, Alkenylierungs-, Alkinylierungs- oder Acylierungsmittel eignen sich z. B. die entsprechenden Chloride, Bromide oder Jodide der Formeln R¹-X bzw. R³-X (worin R¹ bzw. R³ von H verschieden sind), als Acylierungsmittel auch die Anhydride der Formel (R³)₂O (R³ = Acyl). Diese Umsetzungen erfolgen zweckmäßig in Gegenwart oder Abwesenheit eines inerten Lösungsmittels bei Temperaturen zwischen etwa -20 und etwa 200°, vorzugsweise zwischen 0 und 150°. Als Lösungsmittel eignen sich die oben angegebenen. Der Zusatz einer Base bei der Reaktion ist zweckmäßig. Als Basen eignen sich z. B. Alkalimetall-oder Erdalkalimetallhydroxide, -carbonate, -alkoholate oder -hydride, wie Natrium- oder Kaliumhydroxid, -carbonat, -methylat, ethylat oder -hydrid, ferner, insbesondere für die Acylierung, auch sekundäre oder tertiäre Amine, z. B. Triethylamin oder Pyridin.

Alkyliert man eine Verbindung der Formel I, worin R¹ = R³ = H sind, so wird zunächst der Rest R¹ = Alkyl eingeführt; daher sind Verbindungen der Formel I mit R¹ = Alkyl und R³ = H sowie mit R¹ = R³ = Alkyl durch nachträgliche Alkylierung gut erhältlich.

Eine Base der Formel I kann mit einer Säure in das zugehörige Säureadditionssalz überführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z. B. Pikrate, können zur Aufreinigung der Verbindungen der Formel I verwendet werden.

Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet. Als Trennmittel für basische Verbindungen der Formel I eignen sich z. B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und ihrer physiologisch unbedenklischen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks-und/oder Aromastoffe enthalten. Sie können, falls erwünscht, auch einen oder mehrere weitere Wirkstoffe enthalten, z. B. ein oder mehrere Vitamine.

Die Verbindungen der Formel I können bei der Bekämpfung von Krankheiten, insbesondere von Herzinsuffizienz, sowie bei der therapeutischen Behandlung des menschlichen oder tierischen Körpers verwendet werden.

Dabei werden die erfindungsgemäßen Substanzen in der Regel in Analogie zu bekannten positiv inotrop wirksamen Substanzen wie Amrinon verabreicht, vorzugsweise in Dosierungen zwischen etwa 1 und 100 mg, insbesondere zwischen 2 und 20 mg pro Dosierungseinheit. Die tägliche Dosierung liegt vorzugsweise zwischen etwa 0,02 und 2 mg/kg Körpergewicht Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":
Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

### Beispiel 1

Eine Lösung von 2,8 g 6-(2-Chlorpropionyl)-2-oxo-1,2,3,4-tetrahydrochinolin (erhältlich aus 1,2,3,4-Tetrahydro-2-oxo-chinolin und 2-Chlorpropionylchlorid nach Friedel-Crafts) in 40 ml Acetonitril wird mit 2,15 g Hydrazinthioameisensäure-O-ethylester versetzt und 2 Std. gekocht. Man konzentriert, kühlt ab, filtriert das erhaltene 5-(2-Oxo-1,2,3,4-tetrahydrochinolin-6-yl)-6-methyl-3,6-dihydro-1,3,4-thiadiazin-2-on ("A") ab und kristallisiert aus Methanol um. F. 278°.

Analog erhält man die nachstehenden 3,6-Dihydro-1,3,4-thiadiazin-2-one:
5-(2-Oxo-1,2,3,4-tetrahydrochinolin-6-yl)-3,6-dimethyl-, F. 214-216°
5-(2-Oxo-1,2,3,4-tetrahydrochinolin-6-yl)-3-ethyl-6-methyl-, F. 168-169°
5-(1-Methyl-2-oxo-1,2,3,4-tetrahydrochinolin-6-yl)-
5-(1-Methyl-2-oxo-1,2,3,4-tetrahydrochinolin-6-yl)-6-methyl-, F. 215-217°
5-(1-Methyl-2-oxo-1,2,3,4-tetrahydrochinolin-6-yl)-3,6-dimethyl-, F. 137-139°
5-(2-Oxo-4,4-dimethyl-1,2,3,4-tetrahydrochinolin-6-yl)-
5-(2-Oxo-4,4-dimethyl-1,2,3,4-tetrahydrochinolin-6-yl)-6-methyl-, F. 297-298°
5-(2-Oxo-4,4-dimethyl-1,2,3,4-tetrahydrochinolin-6-yl)-3,6-dimethyl-, F. 243-244°
5-(1,4,4-Trimethyl-2-oxo-1,2,3,4-tetrahydrochinolin-6-yl)-
5-(1,4,4-Trimethyl-2-oxo-1,2,3,4-tetrahydrochinolin-6-yl)-6-methyl-, F. 241°
5-(1,4,4-Trimethyl-2-oxo-1,2,3,4-tetrahydrochinolin-6-yl)-3,6-dimethyl-
5-(1-Ethyl-4,4-dimethyl-2-oxo-1,2,3,4-tetrahydrochinolin-6-yl)-6-methyl-
5-(1-Isopropyl-4,4-dimethyl-2-oxo-1,2,3,4-tetrahydrochinolin-6-yl)-6-methyl-, F. 193°
5-(1-Acetyl-2-oxo-1,2,3,4-tetrahydrochinolin-6-yl)-
5-(1-Acetyl-2-oxo-1,2,3,4-tetrahydrochinolin-6-yl)-6-methyl-
5-(1-Acetyl-2-oxo-1,2,3,4-tetrahydrochinolin-6-yl)-3,6-dimethyl-
5-(1-Acetyl-2-oxo-4,4-dimethyl-1,2,3,4-tetrahydrochinolin-6-yl)-
5-(1-Acetyl-2-oxo-4,4-dimethyl-1,2,3,4-tetrahydrochinolin-6-yl)-6-methyl-
5-(1-Acetyl-2-oxo-4,4-dimethyl-1,2,3,4-tetrahydrochinolin-6-yl)-3,6-dimethyl-.

### Beispiel 2

Analog Beispiel 1 erhält man aus 1,2,3,4-Tetrahydro-6-(2-chlorpropionyl)-chinolin [erhältlich durch Reaktion von 1-Acetyl-1,2,3,4-tetrahydrochinolin mit 2-Chlorpropionylchlorid/AlCl₃ zu 1-Acetyl-1,2,3,4-tetrahydro-6-(2-chlorpropionyl)-chinolin (daneben das 7-(2-Chlorpropionyl)-isomere, das chromatographisch abgetrennt werden kann) und nachfolgende Abspaltung der Acetylgruppe mit HCl] das 5-(1,2,3,4-Tetrahydrochinolin-6-yl)-6-methyl-3,6-dihydro-1,3,4-thiadiazin-2-on ("B), F. 168°.

Analog erhält man aus 1,2,3,4-Tetrahydro-7-(2-chlorpropionyl)-chinolin das 5-(1,2,3,4-Tetrahydrochinolin-7-yl)-6-methyl-3,6-dihydro-1,3,4-thiadiazin-2-on.

### Beispiel 3

Analog Beispiel 1 erhält man aus 1-Acetyl-1,2,3,4-tetrahydro-6-(2-chlorpropionyl)-chinolin das 5-(1-Acetyl-1,2,3,4-tetrahydrochinolin-6-yl)-6-methyl-3,6-dihydro-1,3,4-thiadiazin-2-on ("C"), F. 207°.

Analog erhält man aus den entsprechenden Tetrahydrochinolinen:
5-(1-Acetyl-1,2,3,4-tetrahydrochinolin-7-yl)-6-methyl-3,6-dihydro-1,3,4-thiadiazin-2-on, F. 174°
5-(1-Acetyl-4,4-dimethyl-1,2,3,4-tetrahydrochinolin-7-yl)-6-methyl-3,6-dihydro-1,3,4-thiadiazin-2-on, F. 166°.

### Beispiel 4

Eine Lösung von 3 g "A" in 40 ml DMF wird unter Eiskühlung mit 0,32 g NaH versetzt und 1 Stunde gerührt. Nach Zugabe von 1,5 ml Ethyljodid wird 2 Stunden bei 20° gerührt. Man dampft ein, versetzt mit Wasser und filtriert das erhaltene 5-(2-Oxo-1,2,3,4-tetrahydrochinolin-6-yl)-3-ethyl-6-methyl-3,6-dihydro-1,3,4-thiadiazin-2-on ab; F. 168-169° (aus Isopropanol).

Analog erhält man durch Alkylierung, Alkenylierung oder Alkinylierung der entsprechenden Verbindungen der Formel I (R¹ = H) die nachstehenden 3,6-Dihydro-1,3,4-thiadiazin-2-one:
5-(2-Oxo-1,2,3,4-tetrahydrochinolin-6-yl)-3-methyl-
5-(2-Oxo-1,2,3,4-tetrahydrochinolin-6-yl)-3-ethyl-
5-(2-Oxo-1,2,3,4-tetrahydrochinolin-6-yl)-3-butyl-
5-(2-Oxo-1,2,3,4-tetrahydrochinolin-6-yl)-6-methyl-3-propyl-
5-(2-Oxo-1,2,3,4-tetrahydrochinolin-6-yl)-3-isopropyl-6-methyl-, F. 195-196°
5-(2-Oxo-1,2,3,4-tetrahydrochinolin-6-yl)-3-butyl-6-methyl-
5-(2-Oxo-1,2,3,4-tetrahydrochinolin-6-yl)-3-isobutyl-6-methyl-
5-(2-Oxo-1,2,3,4-tetrahydrochinolin-6-yl)-3-sek.-butyl-6-methyl-
5-(2-Oxo-1,2,3,4-tetrahydrochinolin-6-yl)-3-tert.-butyl-6-methyl-
5-(2-Oxo-1,2,3,4-tetrahydrochinolin-6-yl)-6-methyl-3-pentyl-
5-(2-Oxo-1,2,3,4-tetrahydrochinolin-6-yl)-3-allyl-6-methyl-
5-(2-Oxo-1,2,3,4-tetrahydrochinolin-6-yl)-6-methyl-3-propargyl-.

### Beispiel 5

Eine Lösung von 3 g "B" in 30 ml Dichlormethan wird mit 0,5 ml Triethylamin, dann tropfenweise unter Rühren mit 0,8 ml Acetylchlorid versetzt. Man rührt noch 1 Std. bei 20°, zersetzt mit Wasser, arbeitet wie üblich auf und erhält "C", F. 207°.

Analog erhält man die nachstehenden 3,6-Dihydro-1,3,4-thiadiazin-2-one:
5-(1-Acetyl-1,2,3,4-tetrahydrochinolin-6-yl)-, F. 228°
5-(1-Formyl-1,2,3,4-tetrahydrochinolin-6-yl)-6-methyl-
5-(1-Propionyl-1,2,3,4-tetrahydrochinolin-6-yl)-6-methyl-, F. 198°
5-(1-Butyryl-1,2,3,4-tetrahydrochinolin-6-yl)-6-methyl-
5-(1-Isobutyryl-1,2,3,4-tetrahydrochinolin-6-yl)-6-methyl-
5-(1-Valeryl-1,2,3,4-tetrahydrochinolin-6-yl)-6-methyl-
5-(1-Isovaleryl-1,2,3,4-tetrahydrochinolin-6-yl)-6-methyl-
5-(1-Pivaloyl-1,2,3,4-tetrahydrochinolin-6-yl)-6-methyl-, F. 173°
5-(1-Benzoyl-1,2,3,4-tetrahydrochinolin-6-yl)-6-methyl-, F. 218°
5-(1-p-Methoxy-benzoyl-1,2,3,4-tetrahydrochinolin-6-yl)-6-methyl-, F. 233°
5-[1-(3,4-Dimethoxy-benzoyl)-1,2,3,4-tetrahydrochinolin-6-yl]-6-methyl-, F. 127°
5-[1-(3,4-Methylendioxy-benzoyl)-1,2,3,4-tetrahydrochinolin-6-yl]-6-methyl-, F. 239°
5-(1-p-Methylthio-benzoyl)-1,2,3,4-tetrahydrochinolin-6-yl)-6-methyl-
5-(1-p-Methylsulfinyl-benzoyl)-1,2,3,4-tetrahydrochinolin-6-yl)-6-methyl-
5-(1-p-Methylsulfonyl-benzoyl)-1,2,3,4-tetrahydrochinolin-6-yl)-6-methyl-
5-(1-Picolinoyl)-1,2,3,4-tetrahydrochinolin-6-yl)-6-methyl-
5-(1-Nicotinoyl)-1,2,3,4-tetrahydrochinolin-6-yl)-6-methyl-, F. 159°
5-(1-Isonicotinoyl)-1,2,3,4-tetrahydrochinolin-6-yl)-6-methyl-, F. 228°
5-(1-Methansulfonyl-1,2,3,4-tetrahydrochinolin-6-yl)-6-methyl-, F. 186°
5-(1-Benzolsulfonyl-1,2,3,4-tetrahydrochinolin-6-yl)-6-methyl-,
5-(1-p-Toluolsulfonyl-1,2,3,4-tetrahydrochinolin-6-yl)-6-methyl-,
5-(1-Formyl-4,4-dimethyl-1,2,3,4-tetrahydrochinolin-6-yl)-6-methyl-
5-(1-Acetyl-4,4-dimethyl-1,2,3,4-tetrahydrochinolin-6-yl)-6-methyl-, F. 199°
5-(1-Propionyl-4,4-dimethyl-1,2,3,4-tetrahydrochinolin-6-yl)-6-methyl-
5-(1-Butyryl-4,4-dimethyl-1,2,3,4-tetrahydrochinolin-6-yl)-6-methyl-
5-(1-Isobutyryl-4,4-dimethyl-1,2,3,4-tetrahydrochinolin-6-yl)-6-methyl-, F. 190°
5-(1-Valeryl-4,4-dimethyl-1,2,3,4-tetrahydrochinolin-6-yl)-6-methyl-
5-(1-Isovaleryl-4,4-dimethyl-1,2,3,4-tetrahydrochinolin-6-yl)-6-methyl-
5-(1-Pivaloyl-4,4-dimethyl-1,2,3,4-tetrahydrochinolin-6-yl)-6-methyl-
5-(1-Benzoyl-4,4-dimethyl-1,2,3,4-tetrahydrochinolin-6-yl)-6-methyl-
5-(1-p-Methoxy-benzoyl-4,4-dimethyl-1,2,3,4-tetrahydrochinolin-6-yl)-6-methyl-, F. 131°
5-[1-(3,4-Dimethoxy-benzoyl)-4,4-dimethyl-1,2,3,4-tetrahydrochinolin-6-yl]-6-methyl-, F. 134°
5-[1-(3,4-Methylendioxy-benzoyl)-4,4-dimethyl-1,2,3,4-tetrahydrochinolin-6-yl]-6-methyl-
5-(1-p-Methylthio-benzoyl)-4,4-dimethyl-1,2,3,4-tetrahydrochinolin-6-yl]-6-methyl-
5-(1-p-Methylsulfinyl-benzoyl)-4,4-dimethyl-1,2,3,4-tetrahydrochinolin-6-yl]-6-methyl-
5-(1-p-Methylsulfonyl-benzoyl)-4,4-dimethyl-1,2,3,4-tetrahydrochinolin-6-yl]-6-methyl-
5-(1-Picolinoyl-4,4-dimethyl-1,2,3,4-tetrahydrochinolin-6-yl)-6-methyl-
5-(1-Nicotinoyl-4,4-dimethyl-1,2,3,4-tetrahydrochinolin-6-yl)-6-methyl-
5-(1-Isonicotinoyl-4,4-dimethyl-1,2,3,4-tetrahydrochinolin-6-yl)-6-methyl-, F. 148°
5-(1-Methansulfonyl-4,4-dimethyl-1,2,3,4-tetrahydrochinolin-6-yl)-6-methyl-
5-(1-Benzolsulfonyl-4,4-dimethyl-1,2,3,4-tetrahydrochinolin-6-yl)-6-methyl-
5-(1-p-Toluolsulfonyl-4,4-dimethyl-1,2,3,4-tetrahydrochinolin-6-yl)-6-methyl-.

### Beispiel 6

Analog Beispiel 1 erhält man mit 7-(2-Chlorpropionyl)-2,3,4,5-tetrahydro-1H-1-benzazepin-2-on (erhältlich aus 2,3,4,5-Tetrahydro-1H-1-benzazepin-2-on und 2-Chlorpropionyl-chlorid nach Friedel-Crafts) das 5-(2-Oxo-2,3,4,5-tetrahydro-1H-1-benzazepin-7-yl)-6-methyl-3,6-dihydro-1,3,4-thiadiazin-2-on ("D"), F. 230°.

Analog erhält man die nachstehenden 3,6-Dihydro-1,3,4-thiadiazin-2-one:
5-(2-Oxo-2,3,4,5-tetrahydro-1H-1-benzazepin-7-yl)-3,6-dimethyl-
5-(2-Oxo-2,3,4,5-tetrahydro-1H-1-benzazepin-7-yl)-3-ethyl-6-methyl-
5-(1-Methyl-2-oxo-2,3,4,5-tetrahydro-1H-1-benzazepin-7-yl)-
5-(1-Methyl-2-oxo-2,3,4,5-tetrahydro-1H-1-benzazepin-7-yl)-6-methyl-
5-(1-Methyl-2-oxo-2,3,4,5-tetrahydro-1H-1-benzazepin-7-yl)-3,6-dimethyl-
5-(2-Oxo-5,5-dimethyl-2,3,4,5-tetrahydro-1H-1-benzazepin-7-yl)-
5-(2-Oxo-5,5-dimethyl-2,3,4,5-tetrahydro-1H-1-benzazepin-7-yl)-6-methyl-
5-(2-Oxo-5,5-dimethyl-2,3,4,5-tetrahydro-1H-1-benzazepin-7-yl)-3,6-dimethyl-
5-(1,5,5-Trimethyl-2-oxo-2,3,4,5-tetrahydro-1H-1-benzazepin-7-yl)-
5-(1,5,5-Trimethyl-2-oxo-2,3,4,5-tetrahydro-1H-1-benzazepin-7-yl)-6-methyl-
5-(1,5,5-Trimethyl-2-oxo-2,3,4,5-tetrahydro-1H-1-benzazepin-7-yl)-3,6-dimethyl-
5-(1-Ethyl-5,5-dimethyl-2-oxo-2,3,4,5-tetrahydro-1H-1-benzazepin-7-yl)-6-methyl-
5-(1-Isopropyl-5,5-dimethyl-2-oxo-2,3,4,5-tetrahydro-1H-1-benzazepin-7-yl)-6-methyl-
5-(1-Acetyl-2-oxo-2,3,4,5-tetrahydro-1H-1-benzazepin-7-yl)-
5-(1-Acetyl-2-oxo-2,3,4,5-tetrahydro-1H-1-benzazepin-7-yl)-6-methyl-
5-(1-Acetyl-2-oxo-2,3,4,5-tetrahydro-1H-1-benzazepin-7-yl)-3,6-dimethyl-
5-(1-Acetyl-2-oxo-5,5-dimethyl-2,3,4,5-tetrahydro-1H-1-benzazepin-7-yl)-
5-(1-Acetyl-2-oxo-5,5-dimethyl-2,3,4,5-tetrahydro-1H-1-benzazepin-7-yl)-6-methyl-
5-(1-Acetyl-2-oxo-5,5-dimethyl-2,3,4,5-tetrahydro-1H-1-benzazepin-7-yl)-3,6-dimethyl-.

### Beispiel 7

Analog Beispiel 1 erhält man aus 7-(2-Chlorpropionyl)-2,3, 4,5-tetrahydro-1H-1-benzazepin [erhältlich durch Reaktion von 1-Acetyl-2,3,4,5-tetrahydro-1H-1-benzazepin mit 2-Chlorpropionylchlorid/AlCl₃ zu 1-Acetyl-2,3,4,5-tetrahydro-7-(2-chlorpropionyl)-1H-1-benzazepin (daneben das 8-(2-Chlorpropionyl)-isomere, das chromatographisch abgetrennt werden kann) und nachfolgende Abspaltung der Acetylgruppe mit HCl] das 5-(2,3,4,5-Tetrahydro-1H-1-benzazepin-7-yl)-6-methyl-3,6-dihydro-1,3,4-thiadiazin-2-on ("E"), F. 142°.

Analog erhält man aus 8-(2-Chlorpropionyl)-2,3,4,5-tetrahydro-1H-1-benzazepin das 5-(2,3,4,5-Tetrahydro-1H-1-benzazepin-8-yl)-6-methyl-3,6-dihydro-1,3,4-thiadiazin-2-on.

### Beispiel 8

Analog Beispiel 1 erhält man aus 1-Methoxycarbonyl-2,34,5-tetrahydro-7-(2-chlorpropionyl)-1H-1-benzazepin (erhältlich durch Reaktion von 2,3,4,5-Tetrahydro-1H-1-benzazepin mit Chlorameisensäuremethylester zu 1-Methoxycarbonyl-2,3,4,5-tetrahydro-1H-1-benzazepin und nachfolgende Reaktion mit 2-Chlorpropionylchlorid/AlCl₃) das 5-(1-Methoxycarbonyl-2,3,4,5-tetrahydro-1H-1-benzazepin-7-yl)-6-methyl-3,6-dihydro-1,3,4-thiadiazin-2-on, F. 182°.

Analog erhält man aus den entsprechenden Tetrahydro-1H-benzazepinen:
5-(1-Ethoxycarbonyl-2,3,4,5-tetrahydro-1H-1-benzazepin-7-yl)-6-methyl-3,6-dihydro-1,3,4-thiadiazin-2-on, F. 162°
5-(1-Acetyl-5,5-dimethyl-2,3,4,5-tetrahydro-1H-1-benzazepin-7-yl)-6-methyl-3,6-dihydro-1,3,4-thiadiazin-2-on.

### Beispiel 9

Analog Beispiel 4 erhält man aus "D" und Ethyljodid 5-(2-Oxo-2,3,4,5-tetrahydro-1H-1-benzazepin-7-yl)-3-ethyl-6-methyl-3,6-dihydro-1,3,4-thiadiazin-2-on.

Analog erhält man durch Alkylierung, Alkenylierung oder Alkinylierung der entsprechenden Verbindungen der Formel I (R¹ = H) die nachstehenden 3,6-Dihydro-1,3,4-thiadiazin-2-one:
5-(2-Oxo-2,3,4,5-tetrahydro-1H-1-benzazepin-7-yl)-3-methyl-
5-(2-Oxo-2,3,4,5-tetrahydro-1H-1-benzazepin-7-yl)-3-ethyl-
5-(2-Oxo-2,3,4,5-tetrahydro-1H-1-benzazepin-7-yl)-3-butyl-
5-(2-Oxo-2,3,4,5-tetrahydro-1H-1-benzazepin-7-yl)-6-methyl-3-propyl-
5-(2-Oxo-2,3,4,5-tetrahydro-1H-1-benzazepin-7-yl)-3-isopropyl-6-methyl-
5-(2-Oxo-2,3,4,5-tetrahydro-1H-1-benzazepin-7-yl)-3-butyl-6-methyl-
5-(2-Oxo-2,3,4,5-tetrahydro-1H-1-benzazepin-7-yl)-3-isobutyl-6-methyl-
5-(2-Oxo-2,3,4,5-tetrahydro-1H-1-benzazepin-7-yl)-3-sek.-butyl-6-methyl-
5-(2-Oxo-2,3,4,5-tetrahydro-1H-1-benzazepin-7-yl)-3-tert.-butyl-6-methyl-
5-(2-Oxo-2,3,4,5-tetrahydro-1H-1-benzazepin-7-yl)-6-methyl-3-pentyl-
5-(2-Oxo-2,3,4,5-tetrahydro-1H-1-benzazepin-7-yl)-3-allyl-6-methyl-
5-(2-Oxo-2,3,4,5-tetrahydro-1H-1-benzazepin-7-yl)-6-methyl-3-propargyl-.

### Beispiel 10

Analog Beispiel 5 erhält man aus "E" das 5-[1-(3,4-Dimethoxybenzoyl)-2,3,4,5-tetrahydro-1H-1-benzazepin-7-yl]-6-methyl-3,6-dihydro-1,3,4-thiadiazin-2-on, F. 207°.

Analog erhält man die nachstehenden 3,6-Dihydro-1,3,4-thiadiazin-2-one:
5-(1-Acetyl-2,3,4,5-tetrahydro-1H-1-benzazepin-7-yl)-
5-(1-Acetyl-2,3,4,5-tetrahydro-1H-1-benzazepin-7-yl)-6-methyl-, F. 184°
5-(1-Formyl-2,3,4,5-tetrahydro-1H-1-benzazepin-7-yl)-6-methyl-
5-(1-Propionyl-2,3,4,5-tetrahydro-1H-1-benzazepin-7-yl)-6-methyl-
5-(1-Butyryl-2,3,4,5-tetrahydro-1H-1-benzazepin-7-yl)-6-methyl-
5-(1-Isobutyryl-2,3,4,5-tetrahydro-1H-1-benzazepin-7-yl)-6-methyl-
5-(1-Valeryl-2,3,4,5-tetrahydro-1H-1-benzazepin-7-yl)-6-methyl-
5-(1-Isovaleryl-2,3,4,5-tetrahydro-1H-1-benzazepin-7-yl)-6-methyl-
5-(1-Pivaloyl-2,3,4,5-tetrahydro-1H-1-benzazepin-7-yl)-6-methyl-
5-(1-Chloracetyl-2,3,4,5-tetrahydro-1H-1-benzazepin-7-yl)-6-methyl-, ölig
5-(1-Glycyl-2,3,4,5-tetrahydro-1H-1-benzazepin-7-yl)-6-methyl-
5-(1-N-Methylaminoacetyl-2,3,4,5-tetrahydro-1H-1-benzazepin-7-yl)-6-methyl-
5-(1-Dimethylaminoacetyl-2,3,4,5-tetrahydro-1H-1-benzazepin-7-yl-6-methyl-
5-(1-Diethylaminoacetyl-2,3,4,5-tetrahydro-1H-1-benzazepin-7-yl)-6-methyl-
5-(1-Benzoyl-2,3,4,5-tetrahydro-1H-1-benzazepin-7-yl)-6-methyl-
5-(1-p-Methoxybenzoyl-2,3,4,5-tetrahydro-1H-1-benzazepin-7-yl)-6-methyl-, F. 231°
5-[1-(3,4-Methylendioxybenzoyl)-2,3,4,5-tetrahydro-1H-1-benzazepin-7-yl]-6-methyl-
5-(1-p-Methylthiobenzoyl-2,3,4,5-tetrahydro-1H-1-benzazepin-7-yl)-6-methyl-
5-(1-p-Methylsulfinylbenzoyl-2,3,4,5-tetrahydro-1H-1-benzazepin-7-yl)-6-methyl-
5-(1-p-Methylsulfonylbenzoyl-2,3,4,5-tetrahydro-1H-1-benzazepin-7-yl)-6-methyl-
5-(1-p-Fluorbenzoyl-2,3,4,5-tetrahydro-1H-1-benzazepin-7-yl)-6-methyl-, F. 237°
5-[1-(2-Methoxy-4-methylthio-benzoyl)-2,3,4,5-tetrahydro-1H-1-benzazepin-7-yl]-6-methyl-, F. 209°
5-(1-Cyclohexylcarbonyl-2,3,4,5-tetrahydro-1H-1-benzazepin-7-yl)-6-methyl-, F. 146°
5-[1-(2-Thenoyl)-2,3,4,5-tetrahydro-1H-1-benzazepin-7-yl]-6-methyl, F. 215°
5-[1-(3-Thenoyl)-2,3,4,5-tetrahydro-1H-1-benzazepin-7-yl]-6-methyl-
5-(1-Picolinoyl-2,3,4,5-tetrahydro-1H-1-benzazepin-7-yl)-6-methyl-
5-(1-Nicotinoyl-2,3,4,5-tetrahydro-1H-1-benzazepin-7-yl)-6-methyl-, F. 230°
5-(1-Isonicotinoyl-2,3,4,5-tetrahydro-1H-1-benzazepin-7-yl)-6-methyl-, F. 207°
5-(1-Methansulfonyl-2,3,4,5-tetrahydro-1H-1-benzazepin-7-yl)-6-methyl-
5-(1-Benzolsulfonyl-2,3,4,5-tetrahydro-1H-1-benzazepin-7-yl)-6-methyl-
5-(1-p-Toluolsulfonyl-2,3,4,5-tetrahydro-1H-1-benzazepin-7-yl)-6-methyl-
5-(1-Methoxycarbonyl-2,3,4,5-tetrahydro-1H-1-benzazepin-7-yl)-6-methyl-, F. 182°
5-(1-Ethoxycarbonyl-2,3,4,5-tetrahydro-1H-1-benzazepin-7-yl)-6-methyl-, F. 162°
5-(1-Propoxycarbonyl-2,3,4,5-tetrahydro-1H-1-benzazepin-7-yl)-6-methyl-,
5-(1-Isopropoxycarbonyl-2,3,4,5-tetrahydro-1H-1-benzazepin-7-yl)-6-methyl-,
5-(1-Butoxycarbonyl-2,3,4,5-tetrahydro-1H-1-benzazepin-7-yl)-6-methyl-,
5-(1-Phenoxycarbonyl-2,3,4,5-tetrahydro-1H-1-benzazepin-7-yl)-6-methyl-, F. 234°
5-(1-Aminocarbonyl-2,3,4,5-tetrahydro-1H-1-benzazepin-7-yl)-6-methyl-,
5-(1-N-Methylaminocarbonyl-2,3,4,5-tetrahydro-1H-1-benzazepin-7-yl)-6-methyl-,
5-(1-N-Ethylaminocarbonyl-2,3,4,5-tetrahydro-1H-1-benzazepin-7-yl)-6-methyl-,
5-(1-N,N-Dimethylaminocarbonyl-2,3,4,5-tetrahydro-1H-1-benzazepin-7-yl)-6-methyl-,
5-(1-N,N-Diethylaminocarbonyl-2,3,4,5-tetrahydro-1H-1-benzazepin-7-yl)-6-methyl-,
5-(1-Piperidinocarbonyl-2,3,4,5-tetrahydro-1H-1-benzazepin-7-yl)-6-methyl-,
5-(1-Formylaminocarbonyl-2,3,4,5-tetrahydro-1H-1-benzazepin-7-yl)-6-methyl-,
5-(1-Acetylaminocarbonyl-2,3,4,5-tetrahydro-1H-1-benzazepin-7-yl)-6-methyl-,
5-(1-Propionylaminocarbonyl-2,3,4,5-tetrahydro-1H-1-benzazepin-7-yl)-6-methyl-,
5-(1-Butyrylaminocarbonyl-2,3,4,5-tetrahydro-1H-1-benzazepin-7-yl)-6-methyl-,
5-(1-Isobutyrylaminocarbonyl-2,3,4,5-tetrahydro-1H-1-benzazepin-7-yl)-6-methyl-,
5-[1-(3-Methoxy-4-(2-hydroxyethoxy)-benzoyl)-2,3,4,5-tetrahydro-1H-1-benzazepin-7-yl]-6-methyl-, F. 221°
5-(1-Methyl-1,2,3,4-tetrahydrochinolin-6-yl)-6-methyl-, F. 177°
5-(1-Isobutoxycarbonyl-1,2,3,4-tetrahydrochinolin-6-yl)-6-methyl-, F. 127°
5-[1-(2-Methoxy-4-methylthio-benzoyl)-1,2,3,4-tetrahydrochinolin-6-yl]-6-methyl-, F. 160°
5-(1-(2-Methoxy-4-methylsulfinyl-benzoyl)-1,2,3,4-tetrahydrochinolin-6-yl]-6-methyl-, F. 138°
5-(1-p-Fluorbenzoyl-1,2,3,4-tetrahydrochinolin-6-yl)-6-methyl-, F. 221°
5-[1-(3-Methoxy-4-methylsulfinyl-benzoyl)-1,2,3,4-tetrahydrochinolin-6-yl]-6-methyl-, F. 147°
5-(1-Methoxycarbonyl-1,2,3,4-tetrahydrochinolin-6-yl)-6-methyl-, F. 175°
5-[1-(3-Phenyl-5-methyl-4-isoxazolyl-carbonyl)-1,2,3,4-tetrahydrochinolin-6-yl]-6-methyl-, F. 192°
5-[1-(2,4-Dimethoxybenzoyl)-1,2,3,4-tetrahydrochinolin-6-yl]-6-methyl-, F. 162°
5-[1-(3,4,5-Trimethoxybenzoyl)-1,2,3,4-tetrahydrochinolin-6-yl]-6-methyl-, F. 144°
5-(1-p-Dimethylaminobenzoyl-1,2,3,4-tetrahydrochinolin-6-yl)-6-methyl-, F. 218°
5-[1-(3-Methoxy-4-(2-methoxyethoxy)-benzoyl)-1,2,3,4-tetrahydrochinolin-6-yl]-6-methyl-, F. 150°
5-(1-Ethoxycarbonyl-1,2,3,4-tetrahydrochinolin-6-yl)-6-methyl-, F. 148°
5-[1-(3-Methoxy-4-(2-hydroxyethoxy)-benzoyl)-1,2,3,4-tetrahydrochinolin-6-yl]-6-methyl-, F. 108°
5-(1-Hydroxyacetyl-1,2,3,4-tetrahydrochinolin-6-yl)-6-methyl-, F. 211°
5-[1-(3-Methoxy-4-(3-dimethylaminopropoxy)-benzoyl)-1,2,3,4-tetrahydrochinolin-6-yl]-6-methyl-, F. 151°
5-[1-(1,5-Dimethyl-3-pyrazolyl-carbonyl)-1,2,3,4-tetrahydrochinolin-6-yl]-6-methyl-, F. 223°
5-(1-Methoxyacetyl-1,2,3,4-tetrahydrochinolin-6-yl)-6-methyl-, F. 156°
5-[1-(2-Methyl-5-thiazolyl-carbonyl)-1,2,3,4-tetrahydrochinolin-6-yl]-6-methyl-, F. 221°
5-(1-Dimethylaminoacetyl-1,2,3,4-tetrahydrochinolin-6-yl)-6-methyl-, F. 209°
5-[1-(1-Imidazolylcarbonyl)-1,2,3,4-tetrahydrochinolin-6-yl]-6-methyl-, F. 180°
5-(1-Diethylaminoacetyl-1,2,3,4-tetrahydrochinolin-6-yl)-6-methyl-, F. 146°
5-(1-Acetyl-1,2,3,4-tetrahydrochinolin-6-yl)-6-ethyl-, F. 168°
5-[1-(3,4-Dimethoxybenzoyl)-1,2,3,4-tetrahydrochinolin-6-yl]-6-ethyl-, F. 253°
5-(1-Isonicotinoyl-1,2,3,4-tetrahydrochinolin-6-yl)-6-ethyl-, F 236°
5-(1-Benzoyl-1,2,3,4-tetrahydrochinolin-6-yl)-6-ethyl-, F. 201°
5-(1-Methoxycarbonyl-1,2,3,4-tetrahydrochinolin-6-yl)-6-ethyl-, F. 174°
5-(1-Ethoxycarbonyl-1,2,3,4-tetrahydrochinolin-6-yl)-6-ethyl-, F. 158°
5-(1-Hydroxyacetyl-1,2,3,4-tetrahydrochinolin-6-yl)-6-ethyl-, F. 228°
5-[1-(3-Methoxy-4-(2-hydroxyethoxy)-benzoyl)-1,2,3,4-tetrahydrochinolin-6-yl]-6-ethyl-, F. 171°
5-(1-Acryloyl-4,4-dimethyl-1,2,3,4-tetrahydrochinolin-6-yl)-6-methyl-, F. 222°
5-(1-Cyclopropylcarbonyl-4,4-dimethyl-1,2,3,4-tetrahydrochinolin-6-yl)-6-methyl-, F. 210°
5-(1-Cyclohexylcarbonyl-4,4-dimethyl-1,2,3,4-tetrahydrochinolin-6-yl)-6-methyl-, F. 183°
5-(1-Methoxyacetyl-4,4-dimethyl-1,2,3,4-tetrahydrochinolin-6-yl)-6-methyl-, F. 172°
5-(1-Methoxycarbonyl-4,4-dimethyl-1,2,3,4-tetrahydrochinolin-6-yl)-6-methyl-, F. 212°
5-(1-Ethoxycarbonyl-4,4-dimethyl-1,2,3,4-tetrahydrochinolin-6-yl)-6-methyl-, F. 182°
5-(1-N,N-Diethylcarbamoyl-4,4-dimethyl-1,2,3,4-tetrahydrochinolin-6-yl)-6-methyl-, F. 108°
5-[1-(1-Methyl-4-piperidinyl-carbonyl)-4,4-dimethyl-1,2,3,4-tetrahydrochinolin-6-yl]-6-methyl-, F. 247°
5-(1-Isobutyryl-4,4-dimethyl-1,2,3,4-tetrahydrochinolin-7-yl)-6-methyl-, F. 186°
5-(1-Cyclopropylcarbonyl-4,4-dimethyl-1,2,3,4-tetrahydrochinolin-7-yl)-6-methyl-, F. 180°
5-(1-Cyclohexylcarbonyl-4,4-dimethyl-1,2,3,4-tetrahydrochinolin-7-yl)-6-methyl-,
5-(1-p-Methoxybenzoyl-4,4-dimethyl-1,2,3,4-tetrahydrochinolin-7-yl)-6-methyl-, F. 193°
5-[1-(3,4-Dimethoxybenzoyl)-4,4-dimethyl-1,2,3,4-tetrahydrochinolin-7-yl)-6-methyl-,
5-[1-(3-Methoxy-4-methylsulfinyl-benzoyl)-4,4-dimethyl-1,2,3,4-tetrahydrochinolin-7-yl)-6-methyl-, F. 134°
5-(1-Isonicotinoyl-4,4-dimethyl-1,2,3,4-tetrahydrochinolin-7-yl)-6-methyl-, F. 152°
5-(1-Methoxycarbonyl-4,4-dimethyl-1,2,3,4-tetrahydrochinolin-7-yl)-6-methyl-, F. 194°
5-(1-Ethoxycarbonyl-4,4-dimethyl-1,2,3,4-tetrahydrochinolin-7-yl)-6-methyl-, F. 82°
5-(1-Acetyl-4,4-diethyl-1,2,3,4-tetrahydrochinolin-6-yl)-6-methyl-, F. 210°
5-(1-Isonicotinoyl-4,4-diethyl-1,2,3,4-tetrahydrochinolin-6-yl)-6-methyl-, F. 183°.

### Beispiel 11

Analog Beispiel 2 erhält man aus den entsprechenden (2-Chlorpropionyl)-1,2,3,4-tetrahydrochinolinen die folgenden 3,6-Dihydro-1,3,4-thiadiazin-2-one:
5-(4,4-Dimethyl-1,2,3,4-tetrahydrochinolin-6-yl)-6-methyl-, F. 201°
5-(4,4-Dimethyl-1,2,3,4-tetrahydrochinolin-7-yl)-6-methyl-, F. 187°.

Die nachstehenden Beispiele betreffen pharmazeutische Zubereitungen, die Verbindungen der Formel I oder ihre Säureadditionssalze enthalten:

### Beispiel A: Tabletten

Ein Gemisch von 1 kg "A", 10 kg Lactose, 6 kg mikrokristalliner Cellulose, 6 kg Kartoffelstärke, 1 kg Polyvinylpyrrolidon, 0,8 kg Talk und 0,1 kg Magnesiumstearat wird in üblicher Weise zu Tabletten gepreßt, derart, daß jede Tablette 10 mg Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

1 kg 5-(2-Oxo-4,4-dimethyl-1,2,3,4-tetrahydrochinolin-6-yl)-6-methyl-3,6-dihydro-1,3,4-thiadiazin-2-on wird in üblicher Weise in Hartgelatinekapseln gefüllt, so daß jede Kapsel 5 mg Wirkstoff enthält.

### Beispiel D: Ampullen

Eine Lösung von 1 kg "C" in 30 l 1,2-Propandiol wird steril filtriert, in Ampullen abgefüllt, unter sterilen Bedingungen lyophilisiert und steril verschlossen. Jede Ampulle enthält 2 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

## Patentansprüche (Patentansprüche für folgende(n) Vertragsstaat(en): AT, BE, CH, DE, FR, GB, IT, LI, NL, SE)

1. Thiadiazinone der Formel I 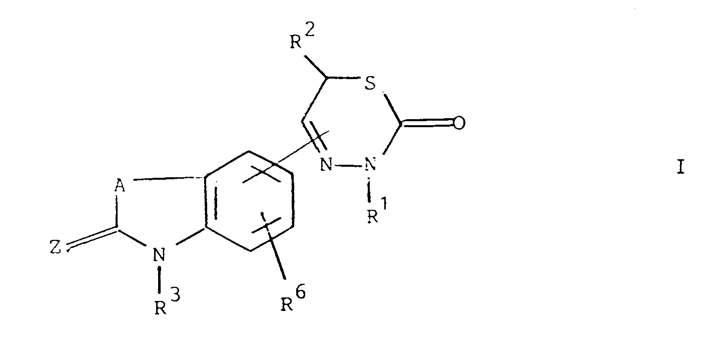 worin
A -CHR⁴-CHR⁵-, -CH₂-CR⁴R⁵-, -CR⁴R⁵-CH₂-, -CHR⁴-CHR⁵-CH₂, -CHR⁴-CH₂-CHR⁵-, -CH₂-CHR⁴-CHR⁵, -CR⁴R⁵-CH₂-CH₂-, -CH₂-CR⁴R⁵-CH₂- oder -CH₂CH₂-CR⁴R⁵-,
R¹, R², R⁴ und R⁵ jeweils H, Alkyl, Alkenyl oder Alkinyl,
R³ Alkenyl, Alkinyl oder Acyl mit bis zu 15 C-Atomen, ausgenommen unsubstituierte Alkanoyl-Reste,
R⁶ H, Alkyl, Alkoxy, OH, F, Cl, Br oder J und
Z (H, H), (H, Alkyl), (Alkyl, Alkyl) oder O
bedeuten,
wobei die Alkyl-, Alkenyl-, Alkinyl- und/oder Alkoxygruppen jeweils bis zu 5 C-Atome enthalten, worin ferner, falls A -CH₂CH₂- und Z O bedeutet, einer der Reste R¹, R², R³ und R⁶ von H verschieden sein muß, sowie deren Salze.

2. a) 5-[1-(3,4-Dimethoxybenzoyl)-1,2,3,4-tetrahydrochinolin-6-yl]-6-methyl-3,6-dihydro-1,3,4-thiadiazin-2-on;
b) 5-(1-Isonicotinoyl-4,4-diethyl-1,2,3,4-tetrahydrochinolin-6-yl)-6-methyl-3,6-dihydro-1,3,4-thiadiazin-2-on.

3. Verfahren zur Herstellung von Thiadiazinonen der Formel I nach Anspruch 1 sowie von deren Salzen, dadurch gekennzeichnet, daß man ein Keton der Formel II 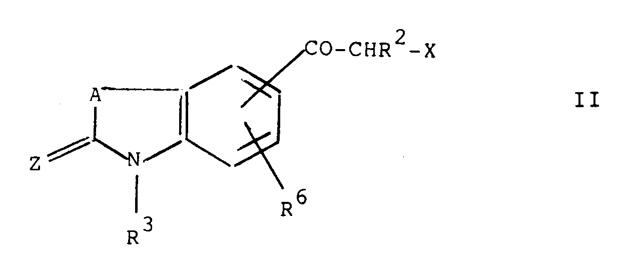 worin
X Cl, Br, J oder eine reaktionsfähig veresterte OH-Gruppe bedeutet und
A, R², R³, R⁶ und Z die angegebenen Bedeutungen haben,
mit einer Verbindung der Formel III
H₂N-NR¹-CS-OR III
worin
R Alkyl mit 1-5 C-Atomen oder ein Äquivalent eines Metall- oder Ammoniumkations
bedeutet und
R¹ die angegebene Bedeutung hat
umsetzt
und/oder daß man gegebenenfalls eine Verbindung der Formel I, worin R¹ und/oder R³ H bedeuten, mit einem Alkylierungs-, Alkenylierungs-, Alkinylierungs- oder Acylierungsmittel behandelt und/oder eine Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verwendung einer Verbindung der Formel I nach Anspruch 1 und/oder eines ihrer physiologisch unbedenklichen Salze zur Herstellung eines Arzneimittels zur Bekämpfung von Krankheiten.

## Patentansprüche (Patentansprüche für folgende(n) Vertragsstaat(en): ES)

1. Verfahren zur Herstellung von Thiadiazinonen der Formel I 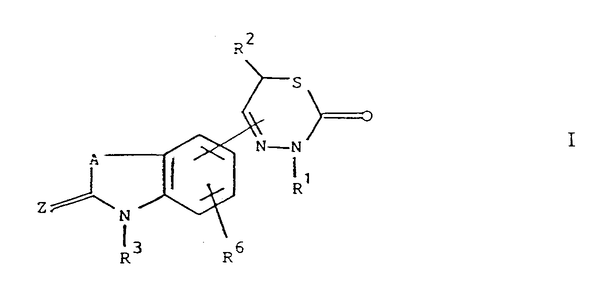 worin
A -CHR⁴-CHR⁵-, -CH₂-CR⁴R⁵, CR⁴R⁵-CH₂-, -CHR⁴-CHR⁵-CH₂ -, CHR⁴-CH₂-CHR⁵-, -CH₂-CHR⁴-CHR⁵-, -CR⁴R⁵-CH₂-CH₂-, -CH₂-CR⁴R⁵-CH₂- oder -CH₂CH₂-CR⁴R⁵-,
R¹, R², R⁴ und R⁵ jeweils H, Alkyl, Alkenyl oder Alkinyl,
R³ Alkenyl, Alkinyl, Acyl mit bis zu 15 C-Atomen, ausgenommen unsubstituierte Alkanoyl-Reste,
R⁶ H, Alkyl, Alkoxy, OH, F, Cl, Br oder I und
Z (H,H), (H,Alkyl), (Alkyl,Alkyl) oder O
bedeuten,
wobei die Alkyl-, Alkenyl-, Alkinyl- und/oder Alkoxygruppen jeweils bis zu 5 C-Atome enthalten, worin ferner, falls A -CH₂-CH₂- und Z O bedeutet, einer der Reste R¹, R², R³ und R⁶ von H verschieden sein muß, sowie von deren Salzen, dadurch gekennzeichnet, daß man ein Keton der Formel II 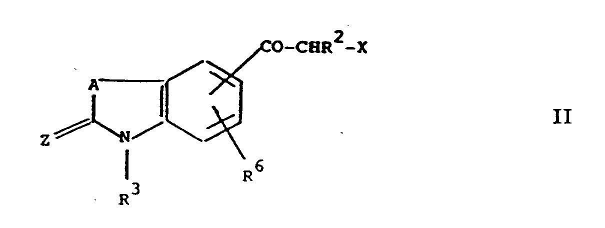 worin
X Cl, Br, I oder eine reaktionsfähig veresterte OH-Gruppe bedeutet und
A, R², R³, R⁶ und Z die angegebenen Bedeutungen haben,
mit einer Verbindung der Formel III
H₂N-NR¹-CS-OR III
worin
R Alkyl mit 1-5 C-Atomen oder ein Äquivalent eines Metall- oder Ammoniumkations
bedeutet und
R¹ die angegebene Bedeutung hat,
umsetzt
und/oder daß man gegebenenfalls eine Verbindung der Formel I, worin R¹ und/oder R³ H bedeuten, mit einem Alkylierungs- oder Acylierungsmittel behandelt und/oder eine Base der Formel I durch Behandeln mit einer Säure in eines ihrer Salze umwandelt.

2. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

## Claims (Claims for the following Contracting State(s): AT, BE, CH, DE, FR, GB, IT, LI, NL, SE)

1. Thiadiazinones of the formula I 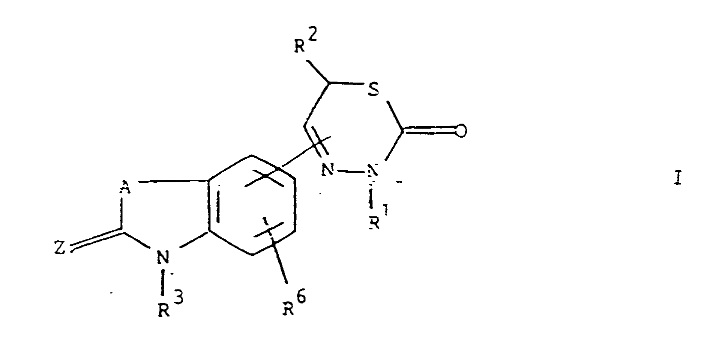 wherein
A is -CHR⁴-CHR⁵-, -CH₂-CR⁴R⁵-, -CR⁴R⁵-CH₂-, -CHR⁴-CHR⁵-CH₂-, -CHR⁴-CH₂-CHR⁵-, -CH₂-CHR⁴-CHR⁵, -CR⁴R⁵-CH₂CH₂, -CH₂-CR⁴R⁵-CH₂ or -CH₂CH₂-CR⁴R⁵-,
R¹, R², R⁴ and R⁵ are each H, alkyl, alkenyl or alkynyl,
R³ is alkenyl, alkynyl or acyl with up to 15 C atoms, with the exception of unsubstituted alkanoyl radicals,
R⁶ is H, alkyl, alkoxy, OH, F, Cl, Br or I and
Z is (H, H), (H, alkyl), (alkyl, alkyl) or O,

2. a) 5-[1-(3,4-Dimethoxybenzoyl)-1,2,3,4-tetrahydroquinolin-6-yl]-6-methyl-3,6-dihydrol,1,3,4-thiadiazin-2-one;
b) 5-(1-isonicotinoyl-4,4-diethyl-1,2,3,4-tetrahydroquinolin-6-yl)-6-methyl-3,6-dihdro-1,3,4-thiadiazin-2-one.

3. Process for the preparation of thiadiazinones of the formula I according to Claim 1 and of salts thereof, characterised in that a ketone of the formula II 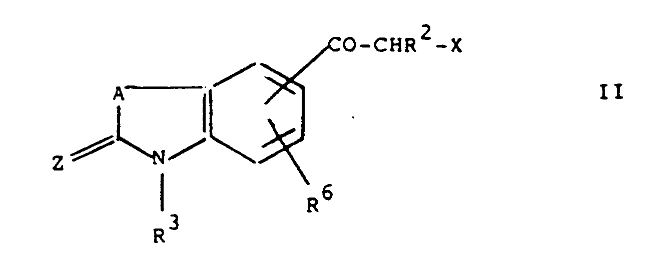 wherein
X is Cl, Br, I or a reactively esterified OH group and
A, R², R³, R⁶ and Z have the meanings given, is reacted with a compound of the formula III
H₂N-NR¹-CS-OR III
wherein
R is alkyl with 1-5 C atoms or one equivalent of a metal or ammonium cation and
R¹ has the meaning given,
and/or in that, if appropriate, a compound of the formula I wherein R¹ and/or R³ is H, is treated with an alkylating, alkenylating, alkynylating or acylating agent, and/or a base of the formula I is converted into one of its salts by treatment with an acid.

4. Process for the preparation of pharmaceutical formulations, characterised in that a compound of the formula I and/or one of its physiologically acceptable salts is brought into a suitable dosage form together with at least one solid, liquid or semi-liquid excipient or auxiliary and if appropriate in combination with one or more other active compound(s).

5. Pharmaceutical formulation, characterised in that it contains at least one compound of the formula I according to Claim 1 and/or one of its physiologically acceptable salts.

6. Use of a compound of the formula I according to Claim 1 and/or one of its physiologically acceptable salts for the preparation of a medicament for combating diseases.

## Claims (Claims for the following Contracting State(s): ES)

1. Process for the preparation of thiadiazinones of the formula I 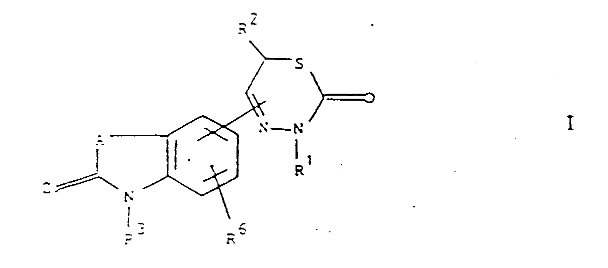 wherein
A is -CHR⁴-CHR⁵-, -CH₂-CR⁴R⁵-, -CR⁴R⁵-CH₂-, -CHR⁴-CHR⁵-CH₂-, -CHR⁴-CH₂-CHR⁵-, -CH₂-CHR⁴-CHR⁵, -CR⁴R⁵-CH₂CH₂-, -CH₂-CR⁴R⁵-CH₂- or -CH₂CH₂-CR⁴R⁵-,
R¹, R², R⁴ and R⁵ are each H, alkyl, alkenyl or alkynyl,
R³ is alkenyl, alkynyl or acyl with up to 15 C atoms, with the exception of unsubstituted alkanoyl radicals,
R⁶ is H, alkyl, alkoxy, OH, F, Cl, Br or I and
Z is (H, H), (H, alkyl), (alkyl, alkyl) or O,
the alkyl, alkenyl, alkynyl and/or alkoxy groups in each case containing up to 5 C atoms, wherein furthermore if A is -CH₂-CH₂- and Z is O, one of the radicals R¹, R², R³ and R⁶ must be other than H, and salts thereof, characterised in that a ketone of the formula II 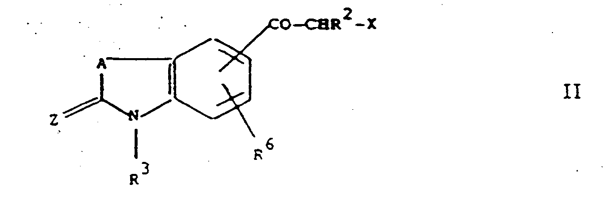 wherein
X is Cl, Br, I or a reactively esterified OH group and
A, R², R³, R⁶ and Z have the meanings given,
is reacted with a compound of the formula III
H₂N-NR¹-CS-OR III
wherein
R is alkyl with 1-5 C atoms or one equivalent of a metal or ammonium cation and
R¹ has the meaning given,
and/or in that, if appropriate, a compound of the formula I wherein R¹ and/or R³ is H, is treated with an alkylating or acylating agent, and/or a base of the formula I is converted into one of its salts by treatment with an acid.

2. Process for the preparation of pharmaceutical formulations, characterised in that a compound of the formula I and/or one of its physiologically acceptable salts is brought into a suitable dosage form together with at least one solid, liquid or semi-liquid excipient or auxiliary and if appropriate in combination with one or more other active compound(s).

## Revendications (Revendications pour l'(les) Etat(s) contractant(s) suivant(s): AT, BE, CH, DE, FR, GB, IT, LI, NL, SE)

1. Thiadiazinone de formule I 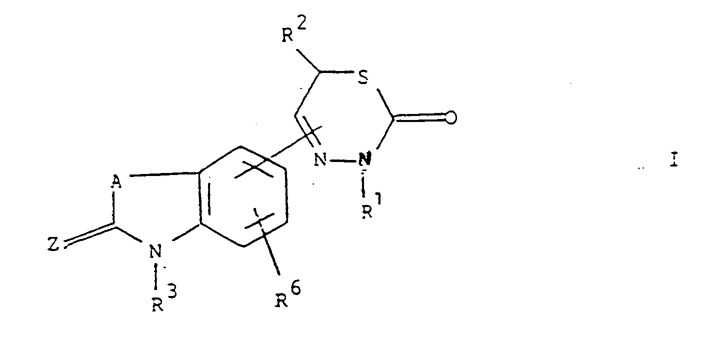 où
A représente
-CHR⁴-CHR⁵-, -CH₂-CR⁴R⁵-, -CR⁴R⁵-CH₂-, -CHR⁴-CHR⁵-CH₂-, -CHR⁴-CH₂-CHR⁵-, -CH₂-CHR⁴-CHR⁵, -CR⁴R⁵-CH₂-CH₂-, -CH₂-CR⁴R⁵-CH₂- ou -CH₂CH₂-CR⁴R⁵-,
R¹, R², R⁴ et R⁵ représentent chacun H, un alkyle, un alcényle ou un alcynyle,
R³ représente un alcényle, un alcynyle ou un acyle ayant jusqu'à 15 atomes de C, à l'exception des restes alcanoyles non substitués,
R⁶ représente H, un alkyle, un alcoxy, OH, F, Cl, Br ou I, et
Z représente (H,H), (H, alkyle), (alkyle, alkyle) ou O.

2. a) 5-[1-(3,4-diméthoxybenzoyl)-1,2,3,4-tétrahydroquinoléine-6-yl]-6-méthyl-3,6-dihydro-1,3,4-thiadiazine-2-one;
b) 5-(1-isonicotinoyl-4,4-diéthyl-1,2,3,4-tétrahydroquinoléine-6-yl)-6-méthyl-3,6-dihydro-1,3,4-thiadiazine-2-one.

3. Procédé pour préparer des thiadiazinones de formule I selon la revendication 1, ainsi que leurs sels, caractérisé en ce que l'on fait réagir une cétone de formule II 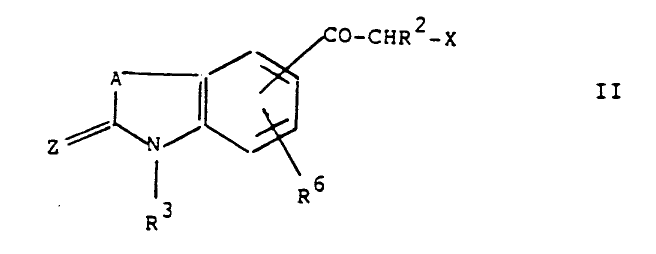 où
X représente Cl, Br, I ou un groupe OH estérifié pouvant réagir, et
A, R², R³, R⁶ et Z ont les significations données précédemment,
avec un composé de formule III
H₂N-NR¹-CS-OR III
où
R représente un alkyle avec de 1 à 5 atomes de C ou un équivalent d'un cation de métal ou d'ammonium
et
R¹ a la signification donnée précédemment
et/ou, le cas échéant, on traite un composé de formule I, où R¹ et/ou R³ représentent H, avec un agent d'alkylation, d'alcénylation, d'alcynylation ou d'acylation, et/ou on transforme une base de formule I en un de ses sels, par traitement avec un acide.

4. Procédé pour produire des préparations pharmaceutiques, caractérisé en ce que l'on met sous une forme de dosage appropriée un composé de formule I et/ou un de ses sels sans inconvénient physiologique, en même temps qu'au moins un support ou additif solide, liquide ou semi-liquide, et, le cas échéant, en combinaison avec une ou plusieurs autres matière(s) active(s).

5. Préparation pharmaceutique, caractérisée en ce qu'elle contient au moins un composé de formule I selon la revendication 1 et/ou un de ses sels sans inconvénient physiologique.

6. Utilisation d'un composé de formule I selon la revendication 1 et/ou d'un de ses sels sans inconvénient physiologique pour produire un médicament pour lutter contre des maladies.

## Revendications (Revendications pour l'(les) Etat(s) contractant(s) suivant(s): ES)

1. Procédé de préparation de thiadiazinones de formule I 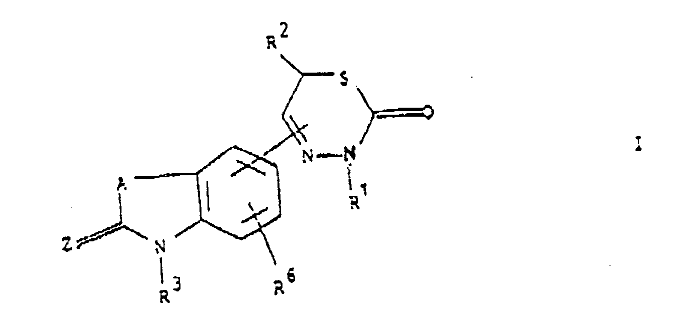 où
A représente -CHR⁴-CHR⁵-, -CH₂-CR⁴R⁵, CR⁴R⁵-CH₂-, -CHR⁴-CHR⁵-CH₂-, -CHR⁴-CH₂-CHR⁵-, -CH₂-CHR⁴-CHR⁵-, -CR⁴R⁵-CH₂-CH₂-, -CH₂-CR⁴R⁵-CH₂- ou -CH₂CH₂-CR⁴R⁵-,
R¹ R² R⁴ et R⁵ représentent chacun H, un alkyle, un alcényle ou un alcynyle,
R³ représente un alcényle, un alcynyle ou un acyle ayant jusqu'à 15 atomes de C, à l'exception des restes alcanoyles non substitués,
R⁶ représente H, un alkyle, un alcoxy, OH, F, Cl, Br ou I, et
Z représente (H,H), (H, alkyle), (alkyle, alkyle) ou O,
où les groupes alkyle, alcényle, alcynyle et/ou alcoxy contiennent chacun jusqu'à 5 atomes de C, et où en outre, si A représente -CH₂-CH₂- et Z représente O, l'un des radicaux R¹, R², R³ et R⁶ doit être différent de H, ainsi que de leurs sels, caractérisé en ce que l'on fait réagir une cétone de formule II 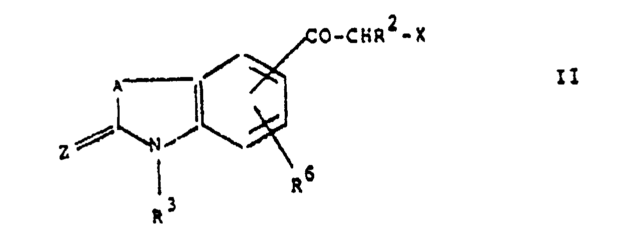 où
X représente Cl, Br, I ou un groupe OH estérifié pouvant réagir, et
A, R², R³, R⁶ et Z ont les significations données précédemment,
avec un composé de formule III
H₂N-NR¹-CS-OR III
où
R représente un alkyle avec de 1 à 5 atomes de C ou un équivalent d'un cation de métal ou d'ammonium
et
R¹ a la signification donnée précédemment ,
et/ou, le cas échéant, on traite un composé de formule I, où R¹ et/ou R³ représentent H, avec un agent d'alkylation ou d'acylation, et/ou on transforme une base de formule I en un de ses sels, par traitement avec un acide.

2. Procédé pour produire des préparations pharmaceutiques, caractérisé en ce que l'on met sous une forme de dosage appropriée un composé de formule I et/ou un de ses sels sans inconvénient physiologique, en même temps qu'au moins un support ou additif solide, liquide ou semi-liquide et, le cas échéant, en combinaison avec une ou plusieurs autres matière(s) active(s).
